# EUROPEAN PATENT SPECIFICATION

(11) **EP 0 902 017 B1**
(45) Date of publication and mention of the grant of the patent: **05.03.2003**
(21) Application number: 98306833.9
(22) Date of filing: 27.08.1998
(51) Int. Cl.: C07D 233/32, C07C 67/03, C07C 69/54, C07C 67/62, C07D 263/04

(54) **Transesterification process**
Verfahren zur Transesterification
Procédé de transestérification

(30) Priority: 29.08.1997 US 57283 P; 12.08.1998 US 133144
(43) Date of publication of application: 17.03.1999
(73) Proprietor: ROHM AND HAAS COMPANY, Philadelphia, Pennsylvania 19106-2399 (US)
(72) Inventor: Weir, William David, Levittown, PA 19056 (US)
(74) Representative: Buckley, Guy Julian

(56) References cited:
- EP-A- 0 433 135
- EP-A- 0 453 638
- EP-A- 0 571 851
- EP-A- 0 650 962
- DE-A- 19 602 035
- US-A- 3 037 006
- US-A- 5 072 027
- JAROSLAV BARTON ET AL.: "Partitioned polymerization,3: On the use of inhibitors partially soluble in the aqueous phase" MAKROMOLEKULARE CHEMIE, MACROMOLECULAR CHEMISTRY AND PHYSICS., vol. 190, no. 4, April 1989, pages 769-775, XP002086582 BASEL CH
- TERRY D.LEE ET AL.: "In Situ Reduction of Nitroxyde Spin Labels with Phenylhydrazine in Deuteriochloroform Solution.A Convenient Method for Obtaining Structural Information on Nitroxydes Using Nuclear Magnetic Resonance Spectroscopy" JOURNAL OF ORGANIC CHEMISTRY., vol. 40, no. 21, 17 October 1975, pages 3145-3147, XP002086583 EASTON US

## Description

This invention relates to a process to prepare monomers, more specifically this invention relates to a transesterification process to prepare monomers.

There are many commercial monomers which are utilized in the preparation of polymers. The monomers are prepared by various processes. There is a need for one process that enables the production of various monomers.

U.S. 4,097,677 discloses a method for preparing dicyclopentenyloxyethyl methacrylate. The disclosed method utilizes acid catalysts, such as sulfuric acid or p-toluenesulfonic acid to catalyze the direct esterification reaction.

U.S. 4,777,265 discloses a method for preparing methacryloyloxyethyl ethylene urea. The disclosed method utilizes chelates of metals such as iron, zinc, titanium, or zirconium as catalysts. Titanium alcoholates are also disclosed as catalysts.

DE-19602035 discloses a transesterification process in which the compound used as the polymerization inhibitor is the 4-hydroxy-2,2,6,6-tetramethylpiperidinyloxyl. The disclosure in Makromol. Chem., 190(1989), 769-775 uses 4-hydroxy-2,2,6,6-tetramethylpiperidinyloxyl radical derivatives as inhibitors of the polymerization of methyl methacrylate under low temperature conditions. However, as US 5,856,562 teaches, the use of such N-oxyl piperidines is not favourable under high temperature conditions.

Magn. Reson. Chem., 34,10 (1996), 743-751 discloses the preparation of 4-hydroxy-2,2,6,6-tetramethyl piperidinyl free radical.

Despite the disclosure of the prior art, there is a continuing need for one process that enables the production of various monomers.

I have surprisingly found that the use of selected catalysts, molar ratios, and conditions provide a process which enables the production of various monomers.

The present invention provides a process comprising:
a) forming a reaction mixture by admixing:
   1) an alcohol selected from the group consisting of hydroxyethyl ethylene urea, ethoxylated cetyl-stearyl alcohol, ethoxylated lauryl-myristyl alcohol, dicyclopentenyloxyethyl alcohol, and hydroxyethyl oxazolidine;
   2) from 10 to 10,000 parts per million based on the alcohol of 4-hydroxy-2,2,6,6-tetramethyl piperidinyl free radical;
   3) methyl methacrylate, wherein the mole ratio of alcohol to methyl methacrylate is from 1:1 to 1:20; and
   4) from 0.1 to 10 mole percent of a catalyst selected from the group consisting of dibutyl tin oxide, reaction products of dibutyl tin oxide with components in the transesterification of various alcohols with alkyl (meth)acrylates; dibutyl tin dimethoxide, reaction products of dibutyl tin dimethoxide with components in the transesterification of various alcohols with alkyl (meth)acrylates; methanolic magnesium methylate; lithium, lithium carbonate, and lithium hydroxide;
b) reacting the alcohol with the methyl methacrylate at a temperature of from 70 to 125°C and a pressure of from 71.6 kgm⁻² to 136.04 kgm⁻² (400 mm Hg to 760 mm Hg);
c) creating a crude product by azeotropically removing a mixture of methyl methacrylate and methanol;
d) optionally adding water to enable recycling of the catalyst;
e) optionally recycling the methyl methacrylate; and
f) optionally distilling the crude product.

The process of this invention enables the production of the following monomers: N - (2- methacryloyloxyethyl) ethylene urea, ethoxylated cetyl-stearyl methacrylate, ethoxylated lauryl-myristyl methacrylate, dicyclopentenyloxyethyl methacrylate, and oxazolidinylethyl methacrylate.

In the process of the invention, starting materials selected from hydroxyethyl ethylene urea, ethoxylated cetyl-stearyl alcohol, ethoxylated lauryl-myristyl alcohol, dicyclopentenyloxyethyl alcohol, and hydroxyethyl oxazolidine are combined with methyl (meth)acrylate in a reaction vessel. The mole ratio of alcohol to (meth)acrylate is typically from 1:1 to 1:20, preferably from 1:1 to 1:15, and more preferably from 1:1.2 to 1:10.

The process of the invention utilizes an Oldershaw distillation column. Suitable Oldershaw columns include a 10 plate X 1" (2.54 cm) diameter column, a 5 plate X 1" diameter column, a 10 plate X 1/2" (1.27 cm) diameter column, and a 32 tray column.

The inhibitor 4-hydroxy-2,2,6,6-tetramethyl piperidinyl free radical, is added to the reaction vessel. The amount of inhibitor added to the reaction vessel is typically from 10 to 10,000 parts per million based on the alcohol charge, preferably from 100 to 5,000 parts per million based on the alcohol charge, and more preferably from 200 to 3,000 parts per million based on the alcohol charge.

A catalyst selected from dibutyl tin oxide, reaction products of dibutyl tin oxide with components in the transesterification of various alcohols with methyl meth(acrylate) or alkyl (meth)acrylates; dibutyl tin dimethoxide, reaction products of dibutyl tin dimethoxide with components in the transesterification of various alcohols with methyl (meth)acrylate or alkyl (meth)acrylates, methanolic magnesium methylate, lithium, lithium carbonate, and lithium hydroxide is added to the reaction vessel. Lithium hydroxide is preferred. The amount of the catalyst added to the reaction vessel is typically from 0.1 to 10 mole percent, preferably from 0.5 to 7 mole percent, and more preferably from 1 to 5 mole percent.

The process of the invention is run at a reaction temperature which ranges from 70-125°C. It is more preferred that the process of the invention be run at from 100-120°C.

The process of the invention is run at pressures ranging from 136.04 kgm⁻² (760 mm Hg) to reduced or elevated pressures. It is preferred that the process of the invention be run at from 71.6 kgm⁻² to 136.04 kgm⁻² (400 mm Hg to 760 mm Hg).

The following abbreviations are used throughout this patent application:
HEEU = hydroxyethyl ethylene urea MMA = methyl methacrylate MAA = methacrylic acid Hg = Mercury MEHQ = p-methoxy phenol DI = deionized PTZ = phenothiazine GLC = gas liquid chromatography °C = degrees centigrade N₂ = nitrogen O₂ = oxygen (meth)acrylate = methacrylate and acrylate cc = cubic centimeter cm = centimeter DEHA = diethylhydroxylamine hr. = hour mm = millimeter ml. = milliliter min. = minute ppm = parts per million MEEU = N - (2- methacryloyloxyethyl) ethylene urea
MEMEU = N-(2-methacryloyloxyethyl)-N'-(methacryloyl) ethylene urea
E20CSA = ethoxylated cetyl-stearyl alcohol
E20CSMA = ethoxylated cetyl-stearyl methacrylate
E23LMA = ethoxylated lauryl-myristyl alcohol
E23LMMA = ethoxylated lauryl-myristyl methacrylate
DCPOEA = dicyclopentenyloxyethyl alcohol
DCPOEM = dicyclopentenyloxyethyl methacrylate
HEOX = hydroxyethyl oxazolidine
OXEMA = oxazolidinylethyl methacrylate
IPOX = 2-isopropyl-3-hydroxyethyl-oxazolidine
DBE = dibasic esters
HPLC = quantitative high performance liquid chromatography

### Example 1: MEEU / H₂O

A mixture of 130.2 grams (1.0 mole) of HEEU, 353.9 grams (3.54 moles) of MMA and 0.2 grams (0.0016 moles) of 4-hydroxy4-hydroxy-2,2,6,6-tetramethyl piperidinyl free radical was added to a 1 liter 4 necked flask equipped with a thermometer, mechanical stirrer, 8% O2 - 92% N2 sparge inlet and a 1 inch diameter - 10 plate Oldershaw column fitted with a distillation head, distillate rate removal-vapor pressure temperature controller, and a graduated distillate receiver. During the dehydration of the batch the mixture was stirred, sparged with 8% O₂ - 92% N₂ at a rate of 3 to 4 ml. min. and heated to reflux under reduced pressure (107.4 kgm⁻²) (600 mm of Hg) for 30 minutes while removing the MMA-water azeotrope. The temperature at the top of the column was 91.7 to 94.5 °C and the temperature in the pot was 94°C. At the conclusion of the dehydration of the batch the solution was cooled to 70°C and to it was added 8.32 grams (0.0334 moles) of dibutyl tin oxide and 2.0 grams (0.02 moles) of MMA. The mixture was stirred, sparged with 8% O₂- 92% N₂ at a rate of 3.8 to 4 ml. / min. and heated to reflux under reduced pressure (107.4 kgm⁻²) (600 mm of Hg) for 4 hours while removing the MMA-methanol of reaction azeotrope. The progress of the reaction was monitored by refractive index analysis of the MMA-methanol of reaction distillate. During the reaction of the batch the temperature at the top of the column was 60.3 to 94.3 °C and the temperature in the pot was 94 to 111 °C. The conversion of HEEU to MEEU was determined to be 102.9% based on the methanol of reaction - MMA azeotrope removal and its analysis for methanol content by refractive index. According to HPLC the reaction mixture, a yellow liquid (311.2 grams), contained 57.2 weight % MEEU, 31.9 weight % MMA, 0.99 weight % HEEU, and 4.82 weight % MEMEU. A mixture of 303.2 grams of the reaction mixture (containing MEEU in MMA) was added to a 1 liter 4 necked flask equipped with a thermometer, mechanical stirrer, graduated addition funnel, 8% O2-92% N2 sparge inlet and a 1 inch diameter - 10 plate Oldershaw column fitted with a distillation head, distillate rate removal-vapor pressure temperature controller, and a graduated distillate receiver. The mixture was stirred, sparged with 8% O₂- 92% N₂ at a rate of 10 ml. / min. and heated to reflux under reduced pressure (44.75 kgm⁻²) (250 mm of Hg) for 35 minutes while removing the MMA-water azeotrope. During the first 24 minutes of the MMA-water azeotrope removal 212.9 grams (11.8 moles) of water was added to the mixture from the graduated addition funnel. During the MMA removal from the batch the temperature at the top of the column was 51.6 to 70.0°C and the temperature in the pot was 61.0 to 75.0°C. At the conclusion of the MMA removal the batch was cooled, decanted, and vacuum filtered through filter paper. According to HPLC the filtrate, 368.2 grams of a yellow product, contained 46.3 weight % MEEU, 0.06 weight % MMA, 0.77 weight % HEEU, 0.16 weight % MAA, and 3.9 weight % MEMEU. By Karl Fisher analysis the product contained 47.6 weight % water.

### Example 2: Isolation of HEEU Process Recycle Catalyst in MMA and Its Use In the Preparation of MEEU / H₂O

The mixture of the water wet solids from the filter cake and decantation of the reaction mixture in EXAMPLE 1, 300.15 grams (3.0 moles) of MMA and 0.198 grams (0.0015 moles) of 4-hydroxy-2,2,6,6-tetramethyl piperidinyl free radical were added to a reactor set up as described in Example 1. The mixture was stirred, sparged with 8% O₂- 92% N₂ at a rate of 3.5 to 4 ml. / min. and heated to reflux under reduced pressure (600 mm of Hg) for 32 minutes while removing the MMA-water azeotrope. During the water removal from the batch the temperature at the top of the column was 77.8 to 93.9°C and the temperature in the pot was 93.0 to 94.0°C. At the conclusion of the dehydration the batch was cooled to ambient 70°C. The mixture (256.7 grams) contained the recycled tin (Sn) containing catalyst and 4-hydroxy-2,2,6,6-tetramethyl piperidinyl free radical in MMA and to it was added 130.0 grams (1.0 mole) of HEEU. The mixture was stirred, sparged with 8% O₂ - 92% N₂ at a rate of 3.8 to 4.2 ml. / min. and heated to reflux under reduced pressure (600 mm of Hg) for 4 hours 25 minutes while removing the MMA-methanol of reaction azeotrope. The progress of the reaction was monitored by refractive index analysis of the MMA-methanol of reaction distillate. During the reaction of the batch the temperature at the top of the column was 60.2 to 92.6°C and the temperature in the pot was 95 to 114°C. The conversion of HEEU to MEEU was determined to be 98.7% based on the methanol of reaction - MMA azeotrope removal and its analysis for methanol content by refractive index. According to HPLC, the reaction mixture, a liquid (305.5 grams), contained 59.8 weight % MEEU, 0.78 weight % MAA, 30.8 weight % MMA, 1.07 weight % HEEU, and 3.5 weight % MEMEU. A mixture of 301.5 grams of the reaction mixture (containing MEEU in MMA) was added to a 1 liter 4 necked flask equipped with a thermometer, mechanical stirrer, graduated addition funnel, 8% 02 - 92% N2 sparge inlet and a 1 inch diameter - 10 plate Oldershaw column fitted with a distillation head, distillate rate removal-vapor pressure temperature controller, and a graduated distillate receiver. The receiver. The mixture was stirred, sparged with 8% O₂- 92% N₂ at a rate of 10-12.5 ml. / min. and heated to reflux under reduced pressure (44.75 kgm⁻²) (250 mm of Hg) for 39 minutes while removing the MMA-water azeotrope. During the first 28 minutes of the MMA-water azeotrope removal 216.0 grams (12.0 moles) of water was added to the mixture from the graduated addition funnel. During the MMA removal from the batch the temperature at the top of the column was 52.9 to 70.7°C and the temperature in the pot was 62.0 to 73.0°C. At the conclusion of the MMA removal the batch was cooled, decanted, and vacuum filtered through filter paper. According to HPLC the filtrate, 374.3 grams of product, contained 47.4 weight % MEEU, no detectable MMA, 0.72 weight % HEEU, 0.29 weight % MAA and 2.8 weight % MEMEU. By Karl Fisher analysis the product contained 48.8 weight % water.

### Example 3: MEEU / H₂O

A mixture of 130.3 grams (1.0 mole) of HEEU, 305.9 grams (3.06 moles) of MMA and 0.2 grams (0.0016 moles) of 4-hydroxy-2,2,6,6-tetramethyl piperidinyl free radical was added to a reactor set up as described in Example 1. During the dehydration of the batch the mixture was stirred, sparged with 8% O₂ - 92% N₂ at a rate of 2 to 8 ml. / min. and heated to reflux under reduced pressure (125.3 kgm⁻²) (700 mm of Hg) for 21 minutes while removing the MMA-water azeotrope. The temperature at the top of the column was 83 to 99.8°C and the temperature in the pot was 100°C. At the conclusion of the dehydration of the batch the solution was cooled to 70°C and to it was added 9.85 grams (0.0334 moles) of dibutyl tin dimethoxide and 17.6 grams (0.175 moles) of MMA. The mixture was stirred, sparged with 8% O₂- 92% N₂ at a rate of 2 to 3 ml. / min. and heated to reflux under reduced pressure (125.3 kgm⁻²) (700 mm of Hg) for 1 hour 55 minutes while removing the MMA-methanol of reaction azeotrope. The progress of the reaction was monitored by refractive index analysis of the MMA-methanol of reaction distillate. During the reaction of the batch the temperature at the top of the column was 64 to 99.1°C and the temperature in the pot was 99 to 115°C. The conversion of HEEU to MEEU was determined to be 99.9% based on the methanol of reaction - MMA azeotrope removal and its analysis for methanol content by refractive index. According to HPLC, the reaction mixture, a yellow liquid (320.1 grams), contained 47.7 weight % MEEU, 27.7 weight % MMA, 1.0 weight % HEEU, and 2.1 weight % MEMEU. A mixture of 319.7 grams of the reaction mixture (containing MEEU in MMA and 20.0 grams {1.11 moles} of water) was added to a 1 liter 4 necked flask equipped with a thermometer, mechanical stirrer, graduated addition funnel, 8% O2 - 92% N2 sparge inlet and a 1 inch diameter - 10 plate Oldershaw column fitted with a distillation head, distillate rate removal-vapor pressure temperature controller, and a graduated distillate receiver. The mixture was stirred, sparged with 8% O₂-92% N₂ at a rate of 12 ml. / min. and heated to reflux under reduced pressure (44.75 kgm⁻²) (250 mm of Hg) for 39 minutes while removing the MMA-water azeotrope. During the first 24 minutes of the MMA-water azeotrope removal 196 grams (10.9 moles of water was added to the mixture from the graduated addition funnel. During the MMA removal from the batch the temperature at the top of the column was 54.2 to 72.6°C and the temperature in the pot was 60 to 73°C. At the conclusion of the MMA removal the batch was cooled, decanted, and vacuum filtered through filter paper. According to HPLC the filtrate, 365.1 grams of a yellow product, contained 39.4 weight % MEEU, no detectable MMA, 0.9 weight % HEEU, 0.02 weight % MAA and 1.6 weight % MEMEU. By Karl Fisher analysis the product contained 48.3 weight % water.

### Example 4: Isolation of HEEU Process Recycle Catalyst in MMA and Its Use In the Preparation of MEEU / H2O

The mixture of the water wet solids from the filter cake and decantation of the reaction mixture in EXAMPLE 3,315.1 grams (3.15 moles) of MMA and 0.198 grams (0.0015 moles) of 4-hydroxy-2,2,6,6-tetramethyl piperidinyl free radical were added to a reactor set up as described in Example 1. The mixture was stirred, sparged with 8% O₂-92% N₂ at a rate of 2.8 to 3.0 ml. / min. and heated to reflux under reduced pressure (125.3 kgm⁻²) (700 mm of Hg) for 31 minutes while removing the MMA-water azeotrope. During the water removal from the batch the temperature at the top of the column was 78.7 to 99.6°C and the temperature in the pot was 93.0 to 99.0°C. At the conclusion of the dehydration the batch was cooled to ambient temperature. To the mixture was added 69.2 grams (0.692 moles) of MMA and it was stirred, sparged with 8% O₂-92% N₂ at a rate of 3.0 ml. / min. and heated to reflux under reduced pressure (125.3 kgm⁻²) (700 mm of Hg) for 39 minutes while removing the MMA-water azeotrope. During the water removal from the batch the temperature at the top of the column was 91.1 to 99.2°C and the temperature in the pot was 99.0°C. At the conclusion of the dehydration the batch was cooled to 63.0°C. The mixture contained the recycled tin (Sn) containing catalyst and 4-hydroxy-2,2,6,6-tetramethyl piperidinyl free radical in MMA and to it was added 130.1 grams (1.0 mole) of HEEU. The mixture was stirred, sparged with 8% O₂- 92% N₂ at a rate of 3.0 ml. / min. and heated to reflux under reduced pressure (125.9 kgm⁻²) (700 mm of Hg) for 4 hours 30 minutes while removing the MMA-methanol of reaction azeotrope. The progress of the reaction was monitored by refractive index analysis of the MMA-methanol of reaction distillate. During the reaction of the batch the temperature at the top of the column was 64.2 to 99.3°C and the temperature in the pot was 100 to 117°C. The conversion of HEEU to MEEU was determined to be 96.6 % based on the methanol of reaction - MMA azeotrope removal and its analysis for methanol content by refractive index. According to HPLC, the reaction mixture, a liquid (355.1 grams), contained 55.8 weight % MEEU, 0.77 weight% MAA, 29.8 weight % MMA, 0.81 weight % HEEU, and 3.5 weight % MEMEU. A mixture of 355.1 grams of the reaction mixture (containing MEEU in MMA and 20.0 grams {1.11 moles} of water) was added to a 1 liter 4 necked flask equipped with a thermometer, mechanical stirrer, graduated addition funnel, 8% O2 - 92% N2 sparge inlet and a 1 inch diameter - 10 plate Oldershaw column fitted with a distillation head, distillate rate removal-vapor pressure temperature controller, and a graduated distillate receiver. The mixture was stirred, sparged with 8% O₂-92% N₂ at a rate of 4 - 10 ml. / min. and heated to reflux under reduced pressure (250 mm of Hg) for 42 minutes while removing the MMA-water azeotrope. During the first 25 minutes of the MMA-water azeotrope removal 196.0 grams (10.88 moles) of water was gradually added to the mixture from the graduated addition funnel. During the MMA removal from the batch the temperature at the top of the column was 53.6 to 72.5°C and the temperature in the pot was 62.0 to 73.0°C. At the conclusion of the MMA removal the batch was cooled, decanted, and vacuum filtered through filter paper. According to HPLC the yellow filtrate, 381.4 grams of product, contained 43.5 weight % MEEU, no detectable MMA, 0.68 weight % HEEU, 0.28 weight % MAA and 3.5 weight % MEMEU. By Karl Fisher analysis the product contained 47.6 weight % water.

### Example 6 - Polyethoxy cetyl-stearyl methacrylate with tetraethylhexyl titanate catalyst:

A mixture of 798.8 grams (0.7 moles) of E20CSA that contained 967 ppm of MEHQ, 500.3 grams (5.0 moles) of MMA, 0.8 grams (0.0065 moles) of MEHQ, 0.2 grams (0.0012 moles) of 4-hydroxy-2,2,6,6-tetramethyl piperidinyl free radical was added to a 2 liter 4-necked flask equipped with a thermometer, mechanical stirrer, 8O₂-92% N₂ sparge inlet and a 1 inch diameter 10 plate Oldershaw column fitted with a distillation head, distillate rate removal-vapor pressure temperature controller, and a graduated distillate receiver. The mixture was stirred, sparged with 8% O₂-92% N₂ at a rate of 32 ml. / min. and heated to reflux under reduced pressure (98.45 kgm⁻²) (550 mm of Hg) for 1.5 hours while removing the MMA-water azeotrope. During the dehydration stage of the batch the temperature at the top of the column was 77.5 to 92°C and the temperature in the pot was 101 to 104°C. At the conclusion of the dehydration of the batch the solution was cooled to 70°C and to it was added 7.9 grams (0.014 moles) of tetraethylhexyl titanate and 83.2 grams (0.83 moles) of MMA. The solution was stirred, sparged with 8% O₂-92% N₂ at a rate of 30 - 35 ml. / min. and heated to reflux under reduced pressure (98.45 kgm⁻²) (550 mm of Hg) for 4 hours while removing the MMA-methanol of reaction azeotrope. The progress of the reaction was monitored by refractive index analysis of the MMA-methanol of reaction distillate. During the reaction stage of the batch the temperature at the top of the column was 57.6 to 91.2°C and the temperature in the pot was 99 to 106°C. The conversion of E20CSA to E20CSMA was determined to be 94.9 % based on the methanol of reaction - MMA azeotrope removal and its analysis for methanol content by refractive index. The MMA was removed in vacuo (pot temperature/pressure of 45 to 109°C / 11.09 kgm⁻² to 8.05 kgm⁻² (62 to 45 mm of Hg)) from the stirred and 8% O2 - 92% N2 sparged mixture. The stirred mixture was cooled to 45°C and 4.10 grams (0.028 moles) of 2-ethyl hexanediol was added. The mixture was stirred and the solution temperature was maintained at 51 to 53°C for 1 hour. To the mixture was added 125.16 grams (1.454 moles) of glacial methacrylic acid. The mixture was stirred for 35 minutes and to it was added 62.6 grams (3.48 moles) of water. The cloud point of the product, an orange liquid, was 52°C. The cloud point was determined by preparing a solution of product in DI water (weight ratio of 1 part product to 99 parts water) and heating the stirred mixture to the point where the mixture became cloudy. The lowest temperature at which the mixture became cloudy was registered as the cloud point.

### Example 6 - Polyethoxy cetyl-stearyl methacrylate with lithium hydroxide catalyst

A mixture of 400.0 grams (0.35 moles) of E20CSA that contained 1,021 ppm of p-methoxy phenol (MEHQ), 250.0 grams (2.5 moles) of methyl methacrylate (MMA), 0.4 grams (0.00323 moles) of MEHQ, and 0.1 grams (0.0006 moles) of 4-hydroxy-2,2,6,6-tetramethyl piperidinyl free radical was added to a 1 liter, 4-necked flask equipped with a thermocouple with temperature readout, mechanical stirrer, 8% O₂ 92% N₂ sparge inlet and a 1 inch diameter 10 plate Oldershaw column fitted with a distillation head, distillate rate removal-vapor pressure temperature controller, and a graduated distillate receiver. The mixture was stirred, sparged with 8% O₂ 92% N₂ at a rate of 7 to 35 ml. / min. and heated to reflux under reduced pressure (98.45 kgm⁻²) (550 mm of Hg) for 0.75 hours while removing the MMA-water azeotrope. During the dehydration stage of the batch the temperature at the top of the column was 65.2 to 85.8°C and the temperature in the pot was 95 to 100° C. At the conclusion of the dehydration of the batch the solution was cooled to 50 to 70°C and to it was added 0.3 grams (0.007 moles) of lithium hydroxide monohydrate and 62.0 grams (0.62 moles) of MMA. The mixture was stirred, sparged with 8% O₂- 92% N₂ at a rate of 7 - 35 ml. / min. and heated to reflux under reduced pressure (98.45 kgm⁻²) (550 mm of Hg) for 2 hours 40 min. while removing the MMA-methanol of reaction azeotrope. The progress of the reaction was monitored by refractive index analysis of the MMA-methanol of reaction distillate. During the reaction stage of the batch the temperature at the top of the column was 58.0 to 87.1°C and the temperature in the pot was 95 to 106°C. The conversion of E20CSA to E20CSMA was calculated to be 115.3 % based on the methanol of reaction-MMA azeotrope removal and its analysis for methanol content by refractive index. The MMA was removed in vacuo (pot temperature / pressure of 55 to 113°C / 21.48 kgm⁻² to 3.58 kgm⁻² (120 to 20 mm of Hg) from the stirred and 8% O2 - 92% N2 sparged mixture. The stirred mixture was cooled to 45°C to afford 423.7 grams of neat E20CSMA.

Some of the neat E20CSMA (141.3 grams) was mixed with 60.5 grams of glacial methacrylic acid for approximately 1/2 hr. at 30 to 45°C. The mixture was polish filtered by vacuum filtration to produce the product mixture of E20CSMA - methacrylic acid. The cloud point of the product, a white - tan liquid, was 51.5 °C.

Some of the neat E20CSMA (141.3 grams) was mixed with 40.4 grams of glacial methacrylic acid for approximately 1/2 hr. at 30 to 45°C. The mixture was stirred for approximately 1/2 hr. and to it was added 20.2 grams of water. The mixture was polish filtered by vacuum filtration to produce the product mixture of E20CSMA - methacrylic acid - water. The cloud point of the product, a white - tan liquid, was 52 to 52.5°C.

For Example 5, the conversion of E20CSA to E20CSMA was 94.9 %. For Example 6, the conversion of E20CSA to E20CSMA was 115.3 %. This data demonstrates that the lithium catalyst is more effective than the titanate catalyst in a transesterification reaction.

### Example 9- MEEU / MMA

A mixture of 260.3 grams (2.0 mole) of HEEU, 1,123.8 grams (11.24 moles) of MMA and 0.398 grams (0.0023 moles) of 4-hydroxy-2,2,6,6-tetramethyl piperidinyl free radical was added to a reactor set up as described in Example 5. The mixture was stirred, sparged with 8% O₂-92% N₂ at a rate of 6 to 7 ml. / min. and heated to reflux under reduced pressure (125.3 kgm⁻²) (700 mm of Hg) for 25 minutes while removing the MMA-water azeotrope. During the dehydration stage of the batch the temperature at the top of the column was 89.8 to 99°C and the temperature in the pot was 100°C. At the conclusion of the dehydration of the batch the solution was cooled to 70°C and to it was added 8.32 grams (0.0335 moles) of dibutyl tin oxide and 93.0 grams (1.0 moles) of MMA. The mixture was stirred, sparged with 8% O₂- 92% N₂ at a rate of 4 - 10 ml. / min. and heated to reflux under reduced pressure (125.3 kgm⁻²) (700 mm of Hg) for 3 hours 38 minutes while removing the MMA-methanol of reaction azeotrope. The progress of the reaction was monitored by refractive index analysis of the MMA -methanol of reaction distillate. During the reaction stage of the batch the temperature at the top of the column was 63.4 to 98.7°C and the temperature in the pot was 100 to 104°C. The conversion of HEEU to MEEU was determined to be 96.7% based on the methanol of reaction - MMA azeotrope removal and its analysis for methanol content by refractive index. According to ,HPLC the product, an orange liquid, contained 28.7 weight % MEEU, 68.0 weight % MMA, 0.45 weight % HEEU, and 0.8 weight % MEMEU.

### Example 10 - DCPOEM

A mixture of 194.0 grams (1.0 mole) of DCPOEA, 120.0 grams (1.2 moles) of MMA, 0.051 grams (0.0004 moles) of MEHQ and 0.049 grams ( 0.0003 moles) of 4-hydroxy-2,2,6,6-tetramethyl piperidinyl free radical was added to a 500 milliliter 4 necked flask equipped with a thermometer, mechanical stirrer, 8% O2 - 92% N2 sparge inlet and a 1 inch diameter - 10 plate Oldershaw column fitted with a distillation head, distillate rate removal-vapor pressure temperature controller, and a graduated distillate receiver. The mixture was stirred, sparged with 8% O₂-92% N₂ at a rate of 8- 10 ml. / min. and heated to reflux under reduced pressure (71.6 kgm⁻²) (400 mm of Hg) for 13 minutes while removing the MMA-water azeotrope. The temperature at the top of the column was 79.3 to 81.4°C and the temperature in the pot was 99 - 103°C. At the conclusion of the dehydration of the batch the solution was cooled to 70°C and to it was added 3.96 grams (0.007 moles) of tetraethylhexyl titanate and 25.0 grams (0.25 moles) of MMA. The mixture was stirred, sparged with 8% O₂ 92% N₂ at a rate of 4 - 7.5 ml. / min. and heated to reflux under reduced pressure (71.6 kgm⁻²) (400 mm of Hg) for 9 hours. During the reaction stage the addition of MMA was made to the batch to help control batch temperature and maintain efficient column operation. The amount of the MMA charges and the elapsed reaction time were: 50 grams (0.5 moles ) of MMA added after 2 1/2 hours of reaction time, followed by 2 hours. of reaction time, followed by the addition of 51 grams (0.51 moles) of MMA and the reaction continuation for 4 1/2 hours. while removing the MMA-methanol of reaction azeotrope. The progress of the reaction was monitored by refractive index analysis of the MMA-methanol of reaction distillate. The temperature at the top of the column was 49.2 to 81.1°C and the temperature in the pot was 99 to 115°C. The conversion of the DCPOEA to DCPOEM was estimated to be 95% based on the methanol of reaction - MMA azeotrope removal and its analysis by refractive index. The MMA was removed in vacuo (pot temperature / pressure of 22 to 88°C / 8.95 kgm⁻² to 3.58 kgm⁻² (50 to 20 mm of Hg) from the stirred and 8% O2 - 92% N2 sparged mixture. The resulting product was isolated (266.1 grams) and analyzed. GLC analysis showed 95.5 area % DCPOEM, 0.63 area % DCPOEA, 0.46 area % MMA and 1.84 area % ethyl hexyl methacrylate (catalyst byproduct).

### Example 11- DCPOEM - distilled grade

A mixture of 291.0 grams (1.5 mole) of DCPOEA, 238.5 grams (2.39 moles) of MMA, 0.074 grams (0.0006 moles) of MEHQ and 0.074 grams ( 0.00043 moles) of 4-hydroxy-2,2,6,6-tetramethyl piperidinyl free radical was added to a reactor set up as described in Example 1. The mixture was stirred, sparged with 8% O₂-92% N₂ and heated to reflux under reduced pressure (71.6 kgm⁻²) (400 mm of Hg) for 16 minutes while removing the MMA-water azeotrope. The temperature at the top of the column was 79.9 to 82.6°C and the temperature in the pot was 96 - 103°C. At the conclusion of the dehydration of the batch the solution was cooled to 70°C and to it was added 5.93 grams (0.0105 moles) of tetraethylhexyl titanate and 10.0 grams (0.1 moles) of MMA. The mixture was stirred, sparged with 8% O₂-92% N₂ and heated to reflux under reduced pressure (71.6 kgm⁻²) (400 mm of Hg) for 5 hrs. During the reaction stage the addition of MMA was made to the batch to help control batch temperature and maintain efficient column operation. The amount of the MMA charges and the elapsed reaction time were:
56.8 grams (0.568 moles ) of MMA added after 2 hrs. of reaction time, followed by 1 hr. of reaction time, followed by the addition of 25.4 grams (0.254 moles) of MMA and 1 hr. of reaction time, followed by the addition of 48.3 grams (0.483 moles) of MMA and the reaction continuation for 1 hr. while removing the MMA-methanol of reaction azeotrope. The progress of the reaction was monitored by refractive index analysis of the MMA-methanol of reaction distillate. The temperature at the top of the column was 49.1 to 82.2°C and the temperature in the pot was 100 to 119°C. The conversion of the DCPOEA to DCPOEM was estimated to be 98% based on the methanol of reaction - MMA azeotrope removal and its analysis by refractive index. A mixture of 0.073 grams (0.00043 moles) of 4-hydroxy-2,2,6,6-tetramethyl piperidinyl free radical and the reaction mixture was fractionally vacuum distilled. The major fraction (290.8 grams), a colorless clear liquid, was obtained during the distillation (vapor temperature range/ pot temperature range / pressure of 126 to 135°C / 154 - 160°C / 0.179 kgm⁻² (1 mm of Hg)). Earlier distillation fractions (73.44 grams) contained 62 - 93.6 area % DCPOEM by GLC analysis and the still bottoms (33.05 grams) was not analyzed. GLC analysis showed that the major fraction contained 96.9 area % DCPOEM, < 0.1 area % DCPOEA, < 0.1 area % MMA and < 0.1 area % ethyl hexyl methacrylate (catalyst byproduct).

### Example 15 - Preparation of 4-Hydroxy-2,2,6,6-tetramethyl N-hydroxy piperidine

To a stirred solution of 126.52 grams (0.735 moles) of 4-hydroxy-2,2,6,6-tetramethyl piperidinyl free radical in 632.64 grams (6.32 moles) of methyl methacrylate was added 65.4 grams (0.73 moles) of N,N-diethyl hydroxylamine. The mixture was stirred at ambient temperature for approximately 5 hrs. and allowed to stand at ambient temperature overnight. The suspension was vacuum filtered. The filter cake was washed with MMA and air dried to yield 88.06 grams of 4-hydroxy-2,2,6,6-tetramethyl N-hydroxy piperidine. The melting point of this product was 55-157 °C.

### Example 16 - Inhibitor Effectiveness

A Base Stock of 660 g of the monomer composition from Example 8, 0.167 mole % (standard amount) DBTO as catalyst and 60.6 ppm (0.2x standard amount) of 4-hydroxy TEMPO (4-hydroxy-2,2,6,6-tetramethyl piperidinyl free radical) as inhibitor was prepared. Four 100 ml one-neck boiling flasks were each set up with a magnetic stir bar and fitted with Y adapter, pot thermometer, West condenser, and heating mantle controlled by a rheostat (set at 65 volts) plugged into a Thermowatch temperature control device. Each flask was charged with a 40 g mixture as follows:
- Flask # 1: Base Stock
- Flask # 2 (Comparative): Base Stock with an extra 300 ppm 4-hydroxy TEMPO added.
- Flask # 3: Base Stock with 300 ppm 4-HT methacrylate added.
- Flask # 4: Base Stock with 300 ppm 4-hydroxy-2,2,6,6-tetramethyl N-hydroxy piperidine added.

All of the above samples were maintained at 92-95°C with agitation and were open to the atmosphere. All samples ran continuously (overnight) at the conditions stated during weekdays, but were cooled to room temperature for the weekend. Samples were tested for soluble polymer by methanol and butyl acrylate ("BA") dilution tests at least twice a day. Methanol and BA dilution tests were conducted using a 1:10 ratio of sample to diluent. The samples passed the soluble polymer tests if clear solution was obtained. The samples failed the soluble polymer tests if haziness or cloudiness was observed. These results are reported below.

| Dilution Test | Flask | Time to Fail (hours) |
|---|---|---|
| Methanol | 1 | 24 |
| | 2 | 132 |
| | 3 | 132 |
| | 4 | 164 |
| BA | 1 | 24 |
| | 2 | 157 |
| | 3 | 132 |
| | 4 | 115.5 |

The above data clearly show that 4-methacrytoyloxy-2,2,6,6-tetramethyl piperidinyl free radical and 4-hydroxy-2,2,6,6-tetramethyl N-hydroxy piperidine are effective inhibitors of unwanted monomer polymerization.

## Claims

1. A process for producing methacrylate monomers which comprises
a) forming a reaction mixture by admixing:
1) an alcohol selected from the group consisting of hydroxyethyl ethylene urea, ethoxylated cetyl-stearyl alcohol, ethoxylated lauryl-myristyl alcohol, dicyclopentenyloxyethyl alcohol, and hydroxyethyl oxazolidine;
2) from 10 to 10,000 parts per million based on the alcohol charge of a free radical inhibitor
3) methyl methacrylate, wherein the mole ratio of alcohol to methyl methacrylate is from 1:1 to 1:20; and
4) from 0.1 to 10 mole percent of a catalyst selected from the group consisting of dibutyl tin oxide, reaction products of dibutyl tin oxide with components in the transesterification of various alcohols with alkyl (meth)acrylates; dibutyl tin dimethoxide, reaction products of dibutyl tin dimethoxide with components in the transesterification of various alcohols with alkyl (meth)acrylates; methanolic magnesium methylate; lithium, lithium carbonate, and lithium hydroxide;
b) reacting the alcohol with the methyl methacrylate at a temperature of from 70 to 125°C and a pressure of from 71.6KgM⁻² to 136.04 KgM⁻² (400 mm Hg to 760 mm Hg);
c) creating a crude product by azeotropically removing a mixture of methyl methacrylate and methanol;
d) optionally adding water to enable recycling of the catalyst;
e) optionally recycling the methyl methacrylate; and
f) optionally distilling the crude product.
**characterized in that** the free radical inhibitor is 4-hydroxy-2,2,6,6-tetramethyl piperidinyl free radical.

2. A process according to claim 1 wherein:
a) the amount of the inhibitor is from 100 to 5,000 parts per million based on the alcohol charge;
b) the mole ratio of alcohol to methyl methacrylate is from 1:1 to 1:15;
c) the amount of the catalyst is from 0.5 to 7 mole percent.

3. A process according to claim 1 wherein:
a) the amount of the inhibitor is from 200 to 3,000 parts per million based on the alcohol charge;
b) the mole ratio of alcohol to methyl methacrylate is from 1:1.2 to 1:10;
c) the amount of the catalyst is from 1 to 5 mole percent; and
d) the reaction is run at a temperature of from 100-120°C.

4. A process according to claim 3 wherein the catalyst is lithium hydroxide.

## Patentansprüche

1. Verfahren zum Herstellen von Methacrylat-Monomeren, umfassend:
a) das Bilden eines Reaktionsgemisches durch Mischen:
1) eines Alkohols, ausgewählt aus der Gruppe, bestehend aus Hydroxyethylethylenharnstoff, ethoxyliertem Cetyl-Stearylalkohol, ethoxyliertem Lauryl-Myristylalkohol, Dicyclopentenyloxyethylalkohol und Hydroxyethyloxazolidin;
2) von 10 bis 10.000 Teilen pro Million, basierend auf der Alkoholbeschickung, eines radikalischen Inhibitors;
3) Methylmethacrylat, wobei das Molverhältnis von Alkohol zu Methylmethacrylat von 1:1 bis 1:20 ist; und
4) von 0,1 bis 10 Molprozent eines Katalysators, ausgewählt aus der Gruppe, bestehend aus Dibutylzinnoxid, Reaktionsprodukten von Dibutylzinnoxid mit Komponenten in der Umesterung von verschiedenen Alkoholen mit Alkyl(meth)acrylaten; Dibutylzinndimethoxid, Reaktionsprodukten von Dibutylzinndimethoxid mit Komponenten in der Umesterung von verschiedenen Alkoholen mit Alkyl(meth)acrylaten: methanolischem Magnesiummethylat; Lithium, Lithiumcarbonat und Lithiumhydroxid;
b) das Umsetzen des Alkohols mit dem Methylmethacrylat bei einer Temperatur von 70 bis 125°C und einem Druck von 71,6 KgM⁻² bis 136,04 KgM⁻² (400 mm Hg bis 760 mm Hg);
c) das Erzeugen eines Rohprodukts durch azeotropes Entfernen eines Gemisches von Methylmethacrylat und Methanol;
d) gegebenenfalls das Zugeben von Wasser, um das Wiedergewinnen des Katalysators zu ermöglichen;
e) gegebenenfalls das Wiedergewinnen des Methylmethacrylats; und
f) gegebenenfalls das Destillieren des Rohprodukts;
**dadurch gekennzeichnet, daß** der radikalische Inhibitor das freie Radikal 4-Hydroxy-2,2,6,6-tetramethylpiperidinyl ist.

2. Verfahren nach Anspruch 1, wobei:
a) die Menge des Inhibitors von 100 bis 5.000 Teilen pro Million, basierend auf der Alkoholbeschickung, ist;
b) das Molverhältnis von Alkohol zu Methylmethacrylat von 1:1 bis 1:15 ist;
c) die Menge des Katalysators von 0,5 bis 7 Molprozent ist.

3. Verfahren nach Anspruch 1, wobei:
a) die Menge des Inhibitors von 200 bis 3.000 Teilen pro Million, basierend auf der Alkoholbeschickung, ist;
b) das Molverhältnis von Alkohol zu Methylmethacrylat von 1:1,2 bis 1:10 ist;
c) die Menge des Katalysators von 1 bis 5 Molprozent ist; und
d) die Reaktion bei einer Temperatur von 100 bis 120°C durchgeführt wird.

4. Verfahren nach Anspruch 3, wobei der Katalysator Lithiumhydroxid ist.

## Revendications

1. Procédé pour produire des méthacrylates monomères, qui comprend
a) la formation d'un mélange réactionnel par mélange:
1) d'un alcool choisi dans le groupe constitué par l'hydroxyéthyléthylène-urée, l'alcool cétylstéarylique éthoxylé, l'alcool laurylmyristylique éthoxylé, l'alcool dicyclopentényloxy-éthylique et l'hydroxyéthyloxazolidine ;
2) de 10 à 10 000 parties par million, par rapport à la charge d'alcool, d'un inhibiteur de radicaux libres ;
3) de méthacrylate de méthyle, le rapport en moles de l'alcool au méthacrylate de méthyle étant de 1:1 à 1:20 ; et
4) de 0,1 à 10 % en moles d'un catalyseur choisi dans le groupe constitué par l'oxyde de dibutylétain, les produits de la réaction de l'oxyde de dibutylétain avec des composants de la transestérification de différents alcools avec des (méth)acrylates d'alkyle ; le diméthoxyde de dibutylétain, les produits de la réaction du diméthoxyde de dibutylétain avec des composants de la transestérification de différents alcools avec des (méth)acrylates d'alkyle; le méthylate de magnésium méthanolique; le lithium, le carbonate de lithium et l'hydroxyde de lithium ;
b) la réaction de l'alcool avec le méthacrylate de méthyle à une température de 70 à 125°C et sous une pression de 71,6 kg.m⁻² à 136,04 kg.m⁻² (400 mm de Hg à 760 mm de Hg);
c) la création d'un produit brut par élimination azéotrope d'un mélange de méthacrylate de méthyle et de méthanol ;
d) éventuellement, l'addition d'eau pour permettre le recyclage du catalyseur ;
e) éventuellement, le recyclage du méthacrylate de méthyle ; et
f) éventuellement, la distillation du produit brut,
**caractérisé en ce que** l'inhibiteur de radicaux libres est le radical libre 4-hydroxy-2,2,6,6-tétraméthylpipéridyle.

2. Procédé selon la revendication 1, dans lequel:
a) la quantité de l'inhibiteur est de 100 à 5 000 parties par million par rapport à la charge d'alcool ;
b) le rapport en moles de l'alcool au méthacrylate de méthyle est de 1:1 à 1:15;
c) la quantité du catalyseur est de 0,5 à 7 % en moles.

3. Procédé selon la revendication 1, dans lequel:
a) la quantité de l'inhibiteur est de 200 à 3 000 parties par million par rapport à la charge d'alcool ;
b) le rapport en moles de l'alcool au méthacrylate de méthyle est de 1:1,2 à 1:10;
c) la quantité du catalyseur est de 1 à 5 % en moles ; et
d) la réaction est mise en oeuvre à une température de 100 à 120°C.

4. Procédé selon la revendication 3, dans lequel le catalyseur est l'hydroxyde de lithium.
